Europäisches Patentamt

European Patent Office

Office européen des brevets

(11) Publication number: 0 194 626
A2

# EUROPEAN PATENT APPLICATION

(21) Application number: 86103143.3

(22) Date of filing: 08.03.86

(51) Int. Cl.4: C12N 15/00 , C07K 15/00

(30) Priority: 15.03.85 US 712558

(43) Date of publication of application:
17.09.86 Bulletin 86/38

(84) Designated Contracting States:
DE GB

(71) Applicant: Albino, Anthony P.
65 West 90 Street
New York New York 10024(US)
Applicant: Silagi, Selma
21-36 33 Road
Long Island City New York 11106(US)
Applicant: Kantor, Raphe R.S.
504 East 81 Street
New York New York 10028(US)
Applicant: Graf, Lloyd H.
1161 York Avenue
New York New York 10021(US)
Applicant: Old, Lloyd J.
600 West End Avenue
New York New York 10024(US)
Applicant: Bander, Neil H.
1385 York Avenue
New York New York 10021(US)

(72) Inventor: Albino, Anthony P.
65 West 90 Street
New York New York 10024(US)
Inventor: Silagi, Selma
21-36 33 Road
Long Island City New York 11106(US)
Inventor: Kantor, Raphe R.S.
504 East 81 Street
New York New York 10028(US)
Inventor: Graf, Lloyd H.
1161 York Avenue
New York New York 10021(US)
Inventor: Old, Lloyd J.
600 West End Avenue
New York New York 10024(US)
Inventor: Bander, Neil H.
1385 York Avenue
New York New York 10021(US)

(74) Representative: Patentanwälte Schulze Horn und
Hoffmeister
Goldstrasse 36
D-4400 Münster(DE)

(54) Method for transformation and direct selection of transformants in eucaryotic cells.

(57) Direct selection of the desired antigenic protein or prot-einaceous product of transformed cells containing exogenous nucleic acid coding for the antigenic product is achieved using one or more of a panel or pool of monoclonal antibodies which detects or is specific for any such antigenic product.

This method eliminates the need for inclusion of a separate phenotypic genetic marker for selection such as a gene for antibiotic resistance. Such a phenotypic marker could be included however, linked or unlinked to the DNA coding for

the desired antigenic product, to allow for pre-screening se-
lection of clones bearing the genetic marker for the desired
antigenic product.

## METHOD FOR TRANSFORMATION AND DIRECT SELECTION OF TRANSFORMANTS IN EUCARYOTIC CELLS

This application is continuation-in-part of copending application Serial No. 606,986 filed May 4, 1984.

This work was supported in part by United States Government funding grants CA 31937 and CA 10095 from National Cancer Institute. Therefore, the government has certain rights in this invention.

This invention concerns recombinant DNA technology wherein exogeneous DNA coding for an antigenic substance is incorporated and expressed in eucaryotic cells. Selection for these transformed cells is effected directly by the use of antibody such as a panel of monoclonal antibodies for selecting out the transformed cells in a screening process.

### Summary

Exogenous or foreign nucleic acid sequences such as found in DNA or RNA coding for specific proteins such as surface cell antigens are transferred into recipient eucaryotic cells. Screening for these specific proteins by a panel of antibodies such as monoclonal antibodies specific for the transferred human DNA protein product allows for the direct selection of the transformed cells.

### Description

In 1980 Stanley Cohen and Herbert Boyer were awarded a patent (4,237,224) which is hereby incorporated by reference for transformation of bacterial cells by means of a plasmid or viral DNA into which was inserted foreign or exogenous DNA material. This altered replicon could then be used to transform bacterial cells which would then express the foreign DNA and its product.

It was the aim of that invention to use the bacterial cell as a living factory to produce for example, hormones and antibiotics eventually, molecules which it could not produce naturally and which were not produced in sufficient quantity by the "foreign" organism.

In the Cohen and Boyer technique selection for transformants is done by means of another piece of DNA inserted into the replicating plasmid or vector -one for antibiotic resistance for examples, to provide a phenotype useful in selecting transformants. Therefore on medium containing the antibiotic, only the transformed cells survive. One then tested these colonies for proper expression of the desired product. Product might be released into the supernatant growth medium or if in the cell, cells would be broken apart and extracted to yield the product. These tend to be tedious biochemical processes. Thus the need for at least two foreign DNA insertions -or co-transformation, where one piece of DNA codes for the phenotype necessary for the selection process and another codes for the desired protein product.

These transformed cells would multiply to provide multiple genomic copies. The method of Cohen and Boyer represented a method for transfer of genetic material in organisms which did not naturally exchange genetic material.

Many eucaryotic cell products however need extra steps past translation to be biologically effective molecules. These extra steps are lacking in bacteria. Bacterial endotoxin contamination is another obstacle to use of these products in animals of humans.

Recently Axel et al. have patented processes for cotransformation of eucaryotic cells (U.S. Patent No. 4,399,216) which is hereby incorporated by reference. These processes depend on a DNA segment acting as a phenotypic marker for selection of transformed cells. The patent basically covers a method for insertion of foreign DNA in linked or unlinked segments into eucaryotic cells. The linked insertion covers an amplification technique producting the phenotype marker in increasing amount in the selection procedure e.g. by increasing antibiotic concentration in the selection medium one selects for multiple copies of the phenotype DNA marker and therefore may get multiple copies as well of the exogeneous DNA coding for the desired protein product.

These linked or unlinked phenotype genetic markers used for selection of transformants must be present in the Axel et al. method. Axel et al. do not select for a specific desired protein product directly, only through the phenotype marker.

In a marked improvement of the above, the present invention uses one or more of a panel of antibodies such as monoclonal antibodies to screen for the desired protein product thereby obviating the need for an added genetic piece serving as a phenotypic marker. In essence the protein product of the transformed cell is the phenotype marker as well which acts not only as a protein product but as an antigenic marker for selection of transformed cells. Thus there is no need for another phenotypic marker to select transformed cells. Inclusion of the second DNA piece can also be done (as linked or unlinked DNA), for use as a pre-screening selection device, before screening positive clones by the method of the invention.

The invention concerns a new direct selection method for transformants not the indirect method of the prior art.

Since most protein products are antigenic the selection method is an immunological one of wide applicability. Highly specific antibody such as monoclonal antibody (mAb) is preferred.

In 1975 Köhler and Milstein [(1975) Nature (London) 256:495] introduced a procedure for the production of monoclonal antibodies (mAbs) using hybrid cells (hybridomas) which allows the production of almost unlimited quantities of antibodies of precise and reproducible specificity. Conventional antisera, produced by immunizing animals with tumor cells or other antigens, contain a myriad of different antibodies differing in their specificity and properties, whereas hybridomas produce a single antibody with uniform characteristics. The Köhler-Milstein procedure entails the fusion of spleen cells from an immunized animal with an immortal myeloma cell line. From the fused cells (hybridomas), clones are selected that produce antibody of the desired specificity. Each clone continues to produce only that one antibody. As hybridoma cells can be cultured indefinitely (or stored frozen in liquid nitrogen), a constant supply of antibody is assured.

Antibodies are proteins that have the ability to combine with and recognize other molecules, known as antigens. Monoclonal antibodies are no different from other antibodies except that they are very uniform in their properties and recognize only one antigen or a portion of an antigen known as a determinant.

In the case of cells, the determinant recognized is an antigen on or in the cell which reacts with the antibody. It is through these cell antigens that a particular antibody recognizes, i.e. reacts with, a particular kind of cell. Thus the cell antigens are markers by which the cell is identified.

These antigenic markers may be used to observe the normal process of cell differentiation, and to locate abnormalities within a given cell system. The process of differentiation is accompanied by changes in the cell surface antigenic phenotype, and antigens that distinguish cells belonging to distinct differentiation lineages or distinguish cells at different phases in the same differentiation lineage may be observed if the correct antibody is available. Initial recognition of differentiation antigens came about through analysis of surface antigens of T-cell leukemias of the mouse and the description of the TL, Thy-1, and Lyt series of antigens. (Old, Lloyd J., Cancer Research, 41, 361-375, February 1981). The analysis of these T-cell differentiation antigens was greatly simplified by the availability of normal T cells and B cells of mouse and man and is relatively advanced. (See Patents #4,361,549--550; #4,364,932-37 and #4,363,799 concerning mAb to Human T-cell antigens). Little is known about differentiation antigens displayed on normal and neoplastic cells belonging to other lineages.

This is due to the difficulty of obtaining a ready source of the appropriate normal cell type as well as the vagaries of the art of monoclonal antibodies. The preparation of hybrid cell lines can be successful or not depending on such experimental factors as nature of the innoculant, cell growth conditions, hybridization conditions etc. Thus it is not always possible to predict successful hybridoma preparation of one cell line although success may have been achieved with another cell line.

Progress in defining surface antigens on melanocytes was made possible by the recently discovered technique of culturing melanocytes from normal skin (Eisinger, et al., Proc. Nat'l. Acad. Sci. USA, 79:2018 (March 1982) U.S. Patent Application S.N. 469,854. This method provides a renewable source of proliferating cells for the analysis of melanocyte differentiation antigens.

Cell surface antigens of human malignant melanoma identified by mouse monoclonal antibodies have been described. (Dippold et al. Proc. Nat'l. Acad. Sci. USA 77, 6114-6118 (1980) U.S. Patent Application Serial Number 307,060. The melanoma work continues in Houghton et al. (1982) J. Exp. Med. 156:1755 U.S. Patent Application S.N. 445,561 and in Albino et al. (1983) J. Immunol. 131:1595 U.S. Patent Application Serial No. 481,378 filed April 1, 1983 which latter concerns mAb to gp130 (found to be the same as the gp150 of Dippold et al. Supra). Previous work on human renal cancer is found in a co-pending patent application Serial No. 277,814 Monoclonal Antibodies To Cell Surface Antigens of Human Renal Cancer and in Serial No. 474,224. U.S. Patent Applications on Colon - (U.S. Patent application serial number 474,415, bladder - (S.N. 567,066) lung (S.N. 474,225) etc. show the wide range of tissue types covered by mAb panels for screening purposes. The above patent applications and references are hereby incorporated by reference.

Therefore serological and biochemical analysis using mouse monoclonal antibodies (mAb) has identified a number of cell surface molecules on cultured and non-cultured human melanoma and other cells. In addition to components of the major histocompatibility complex, these mAbs have defined a variety of differentiation antigens, which has led to an appreciation of the important of specific antigens as markers associated with particular developmental lineages, stages of differentiation and/or neoplastic versus normal states of cell growth. The detailed structure of most of these antigens, as well as their biologic functions, remain to be elucidated. Clearly isolation of recombinant DNA clones encoding surface antigens of interest would greatly aid in the characterization of these molecules and in the dissection of the regulatory mechanisms that govern the specificity of their expression.

To study the structural and functional nature of these antigens, we have transferred human DNA sequences encoding melanoma surface antigens to recipient B78H1 cells, a clonal population of B-16 mouse melanoma cells highly competent for the exogenous uptake and expression of selectable genes. We employed a transformation strategy analagous to that used by Axel et al. Supra that entails application of mixed coprecipitates containing human melanoma DNA together with a cloned selectable vector, pGCcos3neo. Using a pool of 18 mouse mAbs to human melanoma antigens and a rapid screening assay based on red blood cell rosetting and visual identification in situ of primary transfectants expressing new antigens, we have succeeded in stably transferring to B78H1 cells DNA sequences encoding a sialoglycoprotein of 130,000 molecular weight (Mr) (gp130). This antigen is present on cultured human melanoma lines (40/43), most cultures of astrocytoma (7/10) and to a lesser degree on a wide range of other malignant or normal cell types. Subsequent analysis of secondary transfectants revealed that the transfected gp130 had an identical Mr, isoelectric point, intracellular processing and spatial orientation of surface exposed epitopes as that seen with gp130 from human melanoma donor cells. However, cell surface expression of gp130 on mouse transfectants was 2-4 fold less than on the donor human melanoma cells.

The examples illustrate the method of the invention without limiting it. The method of the invention is widely applicable to transformation and selection of transformants generally. Thus clones incorporating genes coding for human proteins or proteinaceous substances which are antigenic such as interferon, growth hormone and the like can be selected for by this method. Any suitable eucaryotic or prokaryotic cell can serve as the competent cell to accept any plasmid or DNA coding for the desired factor.

PREPARATION OF DNA:

High molecular weight DNAs were prepared from cultured human or mouse melanoma cell lines by methods known in the art as described by Axel et al. Supra. Molecular weights of these DNAs were in excess of 50 kilobases - (Kb) as judged by comparison to coelectrophoresed lambda DNA.

Plasmid pGCcos3neo, a derivative of plasmid pBR327, which contains a fragment derived from pSV2neo (Southern and Berg,) (1982) J. Mol. & Applied Genetics I:327-341 including the bacterial gene neo which encodes an aminoglycoside phosphotransferase (APH) (G. Crouse, NCI, Frederick Cancer Research Facility, Frederick, MD) was grown in E. coli host HB101 with ampicillin selection and chloramphenicol amplification. Plasmid DNA was purified and gradient banded by published methods (Maniatis, Frisch and Sambrook, 1982, Molecular Cloning, CSH).

GENE TRANSFER:

An adaptation of the methods in the art as in Axel et al., Supra involving 16-24 hour exposures of cells to coprecipitated cell and plasmid DNAs in the presence of growth medium was used and is further described in Graf, Kaplan and Silagi, (1984) Somatic cell and Mol. Genetics 10:139-151. Briefly, B78H1 cells were plated 24 hours prior to transfection at 500,000 cells/100mm petrie dish (30

dishes total/experiment). 600 micrograms of high molecular weight human or mouse DNA was mixed with 30-60 micrograms pGCcos3neo and allowed to form calcium-phosphate precipitates. 1 ml of precipitate was added per dish (20 micrograms human DNA plus 1-2 micrograms pGCcos3neo) and the dishes incubated at 37°C for 16 hours. The medium was removed and the plates washed with phosphate buffered saline and then refed with complete medium containing 1-1.5 mg Geneticin/ml. Plates were refed every three days. Vector dependent colonies were evident within 5-8 days and screening of these colonies for the expression of transfected antigens was done approximately 2 weeks after selection. Immunoselection was performed on coded dishes transfected with human or mouse DNA.

TISSUE CULTURE:

Melanoma cell lines were established as described previously, Dippold et al., Supra. Cultures were maintained in Eagle's minimum essential medium supplemented with 2mM glutamine, 1% nonessential amino acids, 100U/ml penicillin, 100 micrograms/ml streptomycin, and 10% fetal bovine serum (FBS).

B78H1 cells, a subclone of B-16 mouse melanoma which has been shown to be a highly competent recipient for gene transfer by coprecipitated DNAs ref. and the oubain resistant B78H1 subclone, H1TGO, were grown at 37°C in humidified atmosphere containing 5% $CO_2$ in Eagle's minimal essential medium containing Earle's salts supplemented with 10% heat-inactivated fetal bovine serum - (MA Bioproducts), 50 microgram/ml each of aureomycin - (Lederle) and Gentamycin (Schering). Transferents for the neo gene were selected in the above medium containing 1.5 mg/ml Geneticin as provided by Gibco Co. (630 microgram/ml of pure antibiotic).

Tissue Culture

The renal cancer cell lines (Ueda et al., J. Exp. Med. 150:564-589 (1979)) and tumor cell lines (Carey, et al. Proc. Nat'l. Aoad. Sci. USA 73:3278-3282 (1976)) have been described. Method for the short-term culture of normal kidney epithelium have also been described (Ueda (supra)). Cultures were maintained in Eagle's minimal essential medium supplemented with 2 mM glutamine, 1% nonessential amino acids, 100 units of penicillin per ml, 1 microgram of streptomycin per ml, and 10% (vol/vol) fetal bovine serum. Cultures were regularly tested for mycoplasma, fungi, and bacteria, and contaminated cultures were discarded.

Immunizations.(BALB/C X C57BL/6) $F_1$ female mice were immunized with established normal or cancer cell lines. For the initial immunization, 1 X $10^7$ renal cancer cells for example were injected subcutaneously without adjuvant. Susbsequent immunizations were carried out at intervals of 3-4 weeks by intraperitoneal innoculation of 1 X $10^7$ cells. Immunized mice were sacrificed 3 days after the last immunization.

Derivation of Mouse Abs.The fusion of immune spleen cells with mouse myeloma MOPC-21 NS/1 cells was performed as described (Dippold et al. (1980) Proc. Nat'l. Acad. Sci. USA 77:6114 and Kohler & Milstein, Nature - (London) 236:495-497 (1975) [Also see Ueda et al. Supra and Ueda et al. Proc. Nat'l. Acad. Sci. USA (1981) 78:5122]. Fused cells (5-8 X $10^5$ in 1 ml of selective medium containing hypoxanthine, aminopterin, and theymidine were added to wells of tissue culture plates - (Costar no. 3524, 24 wells pwer plate). Hybridoma cultures were subcloned at least three times by limiting dilution on a feeder layer of 1-3 X $10^5$ mouse peritoneal macrophages. Culture supernatants were monitored for antibody activity on a panel of cultured cells. Antibody subclass was determined by double diffusion in agar with anti-Ig heavy chain specific reagents (Bionetics, Kensington, M.D.). Cultures of cloned hybridomas were injected subcutaneously into nu/nu mice - (Swiss background) and were also stored in liquid nitrogen. Sera from mice with progressively growing tumors were collected, stored at -70°C, and used for serological and biochemical characterization.

SEROLOGICAL PROCEDURES:

Anti-IgG (IgG-MHA) indicator cells were prepared by conjugating the immunoglubulin fraction of rabbit anti-mouse Ig (Dako, Copenhagen) to human O erythrocytes with 0.01% chromium chloride. Wash O RBC once with cold Veronal Buffer (VBM) with graduated centrifugation:

5 min at 1,000 rpm

5 min at 1,500 rpm

5 min at 2,000 rpm

Wash RBC two times more with cold saline with graduated centrifugation. Resuspend pellet in an equal volume of saline. Then while gently vortexing, add a volume of rabbit anti-mouse serum or goat anti-mouse serum, protein A or, in general, serum reactive to mouse Ig equal to the volume of packed RBC. The PA is always at 1mg/ml. Therefore, there is a 1:1:1 ratio of RBC:saline:PA. The Protein A is added slowly. Dilute the 1% $CrCl_3$ solution of 0.01% with saline at 20C. Stock is kept in a dark bottle. Again, while mixing slowly, add dropwise a volume of 0.01% $CrCl_3$ that is equal to 10 times the volume of packed RBC (e.g., if 0.25ml was the volume of RBC then the volume of $CrCl_3$ added would be 2,25ml.

Keep this mixture in suspension for 5 min at 20C. Add VBM and wash two times with graduated centrifugation.

Resuspend in VBM as a 10% (v/v) suspension which diluted to 0.2% working dilution with PBS plus 5% FBS or 1% albumin. Stock RBC-PA is 10% solution which is stable at 4°C for 1-2 weeks. Working dilution is 0.2%. Therefore, dilute stock 50 fold when ready to test target cells for absoption of IgG.

Plates containing neo resistent transferents were washed and incubated with a pool of mouse mAbs for 60 min at 20C. The plates were washed two times with phosphate buffered saline and anti-mouse IgG indicator cells were added to the plates and allowed to incubate for 45 min at 20C. The plates were washed two times and scored for colonies binding erythrocytes which could be seen as a red button when observed under a fluorescent light box, or as erythrocytes bound to individual cells of the colony when viewed microscopically.

IMMUNOPRECIPITATION PROCEDURES:

Cells were radiolabeled as follows: i) metabolic incorporation of (34S)methionine (New England Nuclear, 1000 Ci/mmole; 1 Ci = 3.7 X 10 + 10 becquerels) by using 250 microCi in 12 ml of methionine-free minimal essential medium (MEM) containing 1% FBS for 16 hr, and ii) metabolic incorporation of (3H) glucosamine (New England Nuclear; 30-60 Ci/mMole) by using 150 microCi in 12 ml MEM with 10% FBS for the times indicated. Labeled cells were extracted with 0.5% Nonidet P-40 in 0.15 M NaC/ 0.01 M Tris, pH 7.2 (Tris buffer), containing 0.1 mM phenylmethylsulfonyfluoride, and the solution clarified by centrifugation at 100,000 X g and filtered through a 0.22 micrometer filter. Immunoprecipitation was carried out by mixing a portion of the cell extract (5-10 X 10⁶ cpm) with 1 microliter of mAb, 15 microliters of rabbit anti-mouse Ig - (Cappel Laboratories, Cochranville, PA) serum and 500 microliters of 0.1% Nonidet P-40 in Tris buffer with 1% gamma-globulin free FBS. Immune complexes were isolated by using protein-A bound Separose beads (Pharmacia Co.), and the labeled components were detected by SDS/polyacrylamide gel electrophoresis and fluorography as described.

## PULSE-LABELING PROCEDURES:

Cells in logarithmic phase of growth were cultured with 10 ml of methionine-free MEM at 37C for 60 min. This medium was then removed, and duplicate plates of cells were incubated with 2.5 ml of the same medium plus 100-200 microCi (³⁵S)methionine and incubated at 37C for 15 min. The medium was then removed and the plates washed two times with cold MEM, then re-fed with prewarmed MEM plus 10% FBS. After varying chase periods, the cells were washed once in cold phosphate buffered saline (PBS) and lysed iwth 0.5% Nonidet P-40/0.5% deoxycholate in 0.05 M NaCl/0.02 M Tris, ph 7.4, containing 0.2 mM phenyl-methylsulfonylfluoride. The solution was clarified at 10,000 X g and the supernatant was used for immunoprecipitation reacts as described above.

Example I

## EXPRESSION OF GP130 ON THE CELL SURFACE OF B78H1 TRANSFERENTS.

When a mixed coprecipitate of high-molecular weight SK-MEL-131 DNA and the plasmid pGCcos3neo was added to B78H1 mouse melanoma recipients, a fraction of the neo+ colonies surviving in Geneticin containing selective medium were found to express one or more human melanoma cell surface antigens. Identification of these antigen positive colonies was made on-coded dishes containing neo resistant transferants by means of a mixed erythrocyte resetting assay in which cell surface display of some 18 antigens reactive with individual mouse monoclonal antibodies was simultaneously tested. The gene transfer and the rosetting assay were both highly specific, in that colonies that bound erythrocytes to an extent permitting visualization by macroscopic examination were detected only when coprecipitates contained human melanoma DNA and not when mouse melanoma DNA was employed instead. The majority of colonies thus identified were shown, upon clonal isolation, mass culture growth and immunochemical characterization with each of the 18 mAbs individuals to be expressing a human melanoma sialoglycoprotein, gp130. The aggregate frequency of human melanoma DNA-mediated gp130 gene transfer in the three experiments that yielded transferents was on the average of 1.8 X 10⁻⁶ per B78H1 recipient, or on the average of 4.3 X 10⁻⁴ per Geneticin resistant colony; the frequency on control dishes receiving mouse melanoma DNA was less than 5 X 10-7 per B78H1 recipient. (0 gp130 positive neo gene transferent colonies on 17 dishes). Gp 130 is the gp150 of Dippold et al.,Supra. [Albino et al. J. Immunol. 131:1594 (1983)]

## SECONDARY TRANSFER OF GP130 DNA SEQUENCES.

DNA purified form two primary transfectants of B78H1 expressing gp130 as a consequence of transformation with SK-MEL-131 or SK-MEL-141 DNA could transfer gp130 encoding sequences in secondary founds of transfection of B78H1 cells as described above. Secondary transfer of the gp130 gene occurred in 2 of 2 experiments and it was specific in that only DNA of primary gp130 transferents (6 colonies/4.5 X 10⁺⁶ B78H1 cells) and not control mouse DNA (0 colonies/2 X 10⁺⁶ B78H1 cells) transfers the gp130 positive phenotype.

## RADIOIMMUNOPRECIPITATION ANALYSIS

Radioimmunoprecipitation experiments revealed the anti-gp130 mAb R23 precipitates a protein of 130 kD from both the human donor melanoma line and from the recipient mouse secondary transfectant line. Two-dimensional gel electrophoresis indicates that the gp130 in both cell types has an identical isoelectric point of 5.5, indicating, therefore, that the gp130 protein transcripts of the transfected gene have been glycosylated to the same extent and degree as in human donor melanoma cells.

## INTRACELLULAR PROCESSING OF THE GP130 MOLECULE IN TRANSFECTANTS.

gp130 positive B78H1 secondary transfectant cells were pulsed with (³⁵S)methionine for 15 min, then chased with nonradioactive medium for increasing periods of time. The cells were washed, lysed, and immunoprecipitated with mAb R23. After a 15-min pulse, the only protein species specifically precipitated by mAb R23 migrated with a Mr of 100,000 (designated pr100g;). After a chase period of 30 min, pr100gp is processed into its final form, migrating as a 130 Kd component. No detectable intermediate species between the precursor form and the end product was detected. The processing of the gp130 in mouse secondary transfectants was identical to that seen in the donor human melanoma line, SK-MEL-131, and identical to that shown in another melanoma line, SK-MEL-28 (Dippold et al. Supra).

Both the pr100gp precursor and the final product, gp130 can be labeled with 3H-D-glucosamine. We have previously shown that the biochemical event responsible for the increase in Mr of the pr100gp precursor to 130,000 Kd is the processing of N-linked oligosaccharide side chains, and that an 80 Kd unglycosylated species is the primary translation product of the encoding mRNA. Albino, A.P., et al. (1983) J. Immunol. 131:1595.

## ABSORPTION ANALYSIS OF THE GP130.

To determine if the transfected cells express gp130 at similar levels as the donor melanoma cells, a quantitative absorption analysis was performed (Dippold, et al. Supra. The expression of gp130 on the cell surface of transfected B78H1 secondary mouse cells was approximately 2-4 fold less than that seen on the donor human melanoma line. Control B78H1 cells did not absorb gp130 antibody reactivity over the range of cell numbers tested.

This example describes the method of stable transfer of sequences encoding a cellular antigenic substance such as a cell surface glycoprotein of 130kD from human melanoma genomic DNA to recipient B78H1 mouse melanoma cells, a specific example of the method. The strategy involved: (1) transformation with total cellular DNA with a cloned selectable gene vector, PGCcos3neo; (2) the use of recipient cells which are highly competent for uptake and expression of exogenous DNA sequences such as B78H1 cells; and - (3) the employment of a rapid immunolgical screening assay that permits visual identification and immediate clonal selection of cells expressing transfected genes.

This approach has operational and genetic advantages over fluorescence activated cell systems (FACS) technology previously used to isolate transfected genes in that (a) it is inexpensive, requiring a minimum of resources; (b) a large number of colonies can be screened in a short period of time; (c) it permits identification and isolation of putative antigen transferents upon initial screening with mAbs, even at very low levels of transfer efficiency; and (d) each colony isolated probably represents an independent transfer event. A genetic advantage of the detection of transferent colonies expressing new surface proteins markers by screening viable colonies in situ, as opposed to methods (e.g., FACS or direct immunoselection) which require multiple rounds of enrichment for antigen expressing cells, is the unambiguous independence of transfer events represented by different colony isolates.

As examples we have used genomic DNA from two independently derived cultured melanomas (SK-MEL-131 and SK-MEL-141) and from a cultured lung carcinoma (SK-LU-6) in transfection experiments with B78H1 cells. The resultant transferents, now having the neo resistent phenotype were screened with a battery of 18 mAbs prepared against melanomas (Dippold et al, Supra), but most of which detect antigens having a broad distribution on malignant tissues. From each of these three genomic DNAs the genetic sequences encoding gp130 were transferred to B78H1 cells. The frequency of primary transferents which were positive for gp130 antigen was approximately 1 in 2-5 X $10^3$, irrespective of the donor DNA used.

Inability thus far to isolate B78H1 transferents for any of the 17 other antigens represented in the the mAb pool other than gp130 may reflect inability of the assay to detect colonies of cells displaying extremely low levels of antigen. However, we cannot at present exclude alternative explanations such as involvement in antigenic phenotypes of multiple genes or genes difficult to transfer, restriction of expression of certain genes due to species differences or inappropriateness of the stage of differentiation represented by B78H1 cells, unsuitability of the B78H1 membrane as a matrix for display of potentially antigenic products made from transferred genes, etc.

Using probes for Alu-type repeat sequences and southern blot analysis, large amounts of human DNA was shown to be integrated in the mouse genome of primary transfectants positive for expression of gp130. However, secondary transfectants did not have detectable Alu-type sequences. Presumably, this indicates that the gp130 related sequences are either unique sequence DNA or has other non-Alu-type repeats. While it is formally possible that the lack of detectable major human repeat groups implies that the mouse transfectants are actually expressing a mouse gp130 homologue, we think it unlikely for the following reasons: (1) five monoclonal antibodies to different epitopes (determinants A,B,C,D, & E) of the gp130 molecule are mouse antibodies [Dippold et al, Supra, but note gp150 is gp 130 as determined in Albino et al. J. Immun.131:1595-1599 (1983)] and each reacts with the transfected gp130 molecule displayed on the surface of B78H1 cells; (2) numerous tests failed to detect either cell surface or intracytoplasmic expression of gp130 related molecules in mouse B78H1 recipient cells; - (3) the frequency of transfer of gp130 gene from human DNA to mouse cells is in the range predicted for a single copy gene; (4) no difference was found in the molecular weight, isoelectric point or intracellular processing of gp130 from human cells and that which was presumably transfected to mouse cells; and (5) gp130 expression in B78H1 cells was absolutely dependent upon exposing B78H1 cells to human DNA. However, our present attempts to molecularly clone the transfected gp130 from mouse cells will allow us to definitely prove the human origin of this molecule and to detect any mouse homologue.

Example II

We have recently succeeded in transferring sequences encoding a renal carcinoma cell surface antigen which is biochemically unrelated to the gp130 molecule to B78H1 cells (Kantor et al., manuscript in preparation).

DNA sequences from a human renal carcinoma cell line, SK-RC-41, coding for a cell surface glycoprotein, gp170/140/30, have been stably transferred to recipient B78H1, a clone of B16 mouse melanoma cells using DNA-mediated gene transfer techniques. Gp170/140/30 has been found on virtually all cultured cells; it is seen in tissues only on kidney glomeruli, thyroid microglands and urothelium. Its molecular structure includes a 170,000, 140,000 and six 30,000 weight (mw) species. Calcium phosphate coprecipitates of DNA from SK-RC-41 and the plasmid vector pGCcos3neo were applied to cultures of B78H1. After selection with Geneticin the cells were analyzed for expression of gp170/140/30 using mouse monoclonal antibodies in a mixed hemadsorption rosette assay (Ueda et al. Proc. Nat'l. Acad. Sci. Supra). Southern blot analysis of 1° transfectants with either Alu or whole human nick-translated· probes revealed large amounts of human sequences while 2° transfectants were found to lack major human repeat elements. Biochemical analysis of gp170/140/30 on 2° transfectants compared to SK-RC-41 revealed 1) 120 fold lower expression 2) slightly different mw of 170,000 and 140,000 polypeptides 3) multiple forms of the 140,000 and 30,000 mw species. These differences suggest either an altered processing of gp170/140/30 or a possible recombination with an homologous murine species.

We are currently engaged in elevating the number of copies, and hence the net cellular expression of introduced human genes by cotransferring them with an amplifiable vector into B78H1 cells. Results of these experiments may provide information about the level at which restrictions on the expression of transferent phenotypes for antigens other than gp130 lie.

It will be obvious to those skilled in the art following the teaching of this invention to use specific antibodies such as monoclonal antibodies to generally select for transformed cells directly since the specific mAb identifies the gene protein product directly. A recent publication used the method of the invention Littman et al. Cell 40:237-246 (February 1985) at page 238, col 1. 1st and second full paragraphs. The authors were taught the method at Sloan-Kettering by the inventors. The authors apply the method to detect a variety of human T lymphocyte cell surface antigens expressed in mouse L cells. The authors observe that rosette-positive clone is never seen in the absence of the transforming DNA, thereby confirming the reliability of the tests.

Thus one can also use single or pooled mAbs to select for transformed cells quickly, e.g. pools of 10, 20 or 100 mAbs or groups of pooled mAbs to select for the expression of the desired foreign antigenic product. Pre-screening using a selectable genetic marker such as one coding for antibiotic resistance is also possible. Multiple copies can also be incorporated as well as several different DNA's coding for different antigens.

## Claims

1. Method for stably incorporating nucleic acid coding for a foreign antigenic substance into a cell wherein expression of said antigenic substance as expressed in the cell is screened for directly which comprises:

a) transforming a competent cell with nucleic acid coding for said antigenic substance;

b) selecting for transformed cells expressing said antigenic substance with a panel of monoclonal antibodies wherein one or more of said monoclonal antibodies is specific for the antigen resulting in an antigen-antibody reaction; and

c) detecting the extent of the antigen-antibody reaction by an immunological test.

2. Method of Claim 1 wherein the antigen is a cell surface antigen.

3. Method of Claim 1 wherein the antigen is a human cell surface antigen.

4. Method of Claim 1 wherein the antigen is a human tumor antigen.

5. Method of Claim 1 wherein the antigen is a human cell surface tumor antigen.

6. Method of Claim 1 wherein the antigen is a proteinaceous antigen.

7. Method of Claim 1 wherein the antigen is a proteinaceous cellular substance.

8. Method of Claim 1 wherein a selectable phenotypic marker is included in the foreign nucleic acid for pre-screening prior to step b).

9. Method of Claim 8 wherein the selectable phenotypic marker encodes resistance to an antibiotic.

10. Method of Claim 1 wherein the immunological test is selected from the group consisting of absorption, immune adherence, ELISA, fluorescent antibody, erythrocyte rosetting, MHA and mixtures thereof.

11. Method of Claim 1 wherein the competent cell is a eucaryotic cell.

12. Method for direct selection of expression of a nucleic acid coding for a human tumor cell surface antigen stably incorporated into a cell which comprises:

a) transforming the cell with human nucleic acid coding for the human tumor cell surface antigen;

b) selecting directly for cells expressing the human tumor cell surface antigen with a panel of monoclonal antibodies wherein one or more of said monoclonal antibodies is specific for the antigenic human tumor cell marker resulting in an antigen-antibody reaction; and

c) detecting the extent of the antigen-antibody reaction by means of an immunological test.

13. Method of Claim 12 wherein a selectable phenotypic marker is included in the foreign nucleic acid for pre-screening prior to step b).

14. Method of Claim 13 wherein the selectable phenotypic marker encodes resistance to an antibiotic.

15. Method of Claim 12 wherein the immunological test is selected from the group consisting of absorption, immune adherence, ELISA, fluorescent antibody, erythrocyte rosetting, MHA and mixtures thereof.

16. Method of Claim 12 wherein the transformed cell is a eukaryotic cell.

17. Method for detection of the expression of a foreign antigen in eucaryotic cells which comprises:

a) stably transforming a competent eucaryotic cell with DNA coding for the foreign antigen;

b) selecting for expression of said antigen with a panel of mAb wherein one or more of the mAb is specific for the antigen thereby resulting in an antigen-antibody reaction; and

c) detecting the extent of the antigen-antibody reaction by an immunological test.

18. Method of Claim 17 wherein the antigen is a cell surface antigen.

19. Method of Claim 17 wherein the antigen is a human cell surface antigen.

20. Method of Claim 17 wherein the antigen is a human tumor antigen.

21. Method of Claim 17 wherein the antigen is a human cell surface tumor antigen.

22. Method of Claim 17 wherein the antigen is a proteinaceous antigen.

23. Method of Claim 17 wherein the antigen is a proteinaceous cellular substance.

24. Method of Claim 17 wherein a selectable phenotypic marker is included in the foreign nucleic acid for pre-screening prior to step b).

25. Method of Claim 17 wherein the selectable phenotypic marker encodes resistance to an antibiotic.

26. Method of Claim 17 wherein the immunological test is selected from the group consisting of absorption, immune adherence, ELISA, fluorescent antibody, erythrocyte roset- ting, MHA and mixtures thereof.

27. Monoclonal antibody as used in a method of screening for transformed cells incorporating exogenous DNA coding for antigenic proteinaceous products.